# EUROPEAN PATENT SPECIFICATION

(11) **EP 3 033 070 B1**
(45) Date of publication and mention of the grant of the patent: **12.12.2018**
(21) Application number: 14750357.7
(22) Date of filing: 08.08.2014
(51) Int. Cl.: A61K 8/34, A61K 8/365, A61K 8/41, A61Q 5/06

(54) **PROCESS FOR TREATING KERATIN FIBRES USING AN AQUEOUS-ALCOHOLIC COMPOSITION COMPRISING AN ORGANIC MONOACID**
VERFAHREN ZUR BEHANDLUNG VON KERATINFASERN UNTER VERWENDUNG EINER WÄSSRIG-ALKOHOLISCHEN ZUSAMMENSETZUNG MIT EINER ORGANISCHEN MONOSÄURE
PROCÉDÉ DE TRAITEMENT DES FIBRES KÉRATINIQUES À L'AIDE D'UNE COMPOSITION À BASE D'EAU-ALCOOL COMPRENANT UN MONOACIDE

(30) Priority: 13.08.2013 FR 1357983
(43) Date of publication of application: 22.06.2016
(73) Proprietor: L'Oréal, 75008 Paris (FR)
(72) Inventor: BIATO, Camila, CEP : 22795-295 Rio de Janeiro - RJ (BR); SILVESTRE, Liliane, CEP:21235-602 Rio de Janeiro - RJ (BR); DAUBRESSE, Nicolas, F-78170 La Celle Saint Cloud (FR)
(74) Representative: Leray, Noelle
(86) International application number: PCT/EP2014/067107
(87) International publication number: WO 2015/022276

(56) References cited:
- WO-A2-2011/003665
- US-A1- 2004 011 373
- US-A1- 2006 045 862
- US-A1- 2012 216 823

## Description

The present invention relates to a process for treating keratin fibres, especially the hair, comprising the application of an aqueous-alcoholic composition comprising a monoacid.

In the hair field, consumers wish to have available compositions which make it possible to introduce a temporary change to their head of hair, while targeting good persistence of the effect produced. In general, it is desired for the change to withstand shampooing for at least fifteen days or even more, depending on the nature of the said change.

Treatments already exist for modifying the colour or shape of the hair and also, to a certain extent, the texture of the hair. One of the treatments known for modifying the texture of the hair consists in combining heat and a composition comprising formaldehyde. This treatment is especially effective for imparting a better appearance to damaged hair and/or for treating long hair and curly hair. The action of formaldehyde is associated with its ability to crosslink proteins by reaction on the nucleophilic sites thereof. The heat used may be that of an iron (flat tongs or crimping iron), the temperature of which may generally be up to 200°C or more. However, it is increasingly sought to avoid the use of such substances, which may prove to be aggressive to the hair and other keratin materials.

Patent application WO 2011/104 282 thus proposed a novel process for semi-permanently straightening the hair, which consists in applying an α-keto acid solution to the hair for 15 to 120 minutes, then drying and, finally, straightening the head of hair with an iron at a temperature of about 200°C. The α-keto acid employed is preferably glyoxylic acid. WO 2011/003 665 discloses a process for straightening hair by using heated smoothing irons and composition comprising pipecolic acid and silicone emulsion DC959 which contains low amount of methanol.

However, it has been noted that glyoxylic acid may not be well tolerated, in particular when the scalp is sensitive and/or irritated. Its volatility, amplified by the use of heat (iron), can also be a problem. Furthermore, the compositions of the prior art may impair the hair and/or impair its colour.

The aim of the invention is to develop a hair treatment process, especially a straightening/relaxing process for straightening/relaxing and/or reducing the volume of the hair efficiently and durably and limiting the degradation of the hair, while at the same time maintaining comfort at the time of application for the user of the composition, but also for the stylist who applies it.

Thus, one subject of the present invention is a process for treating keratin fibres, in particular the hair, comprising the application to the hair of an aqueous-alcoholic composition comprising at least 5% of at least one C1-C7 alcohol and at least one organic monoacid, followed by a straightening/relaxing step using a straightening iron at a temperature of at least 100°C.

The process of the invention allows good straightening/relaxing of keratin fibres while at the same time limiting the degradation of these keratin fibres and maintaining an appreciated working quality, especially without excessive vaporization of the composition at the time of straightening. The keratin fibre treatment process according to the invention also makes it possible to limit the change in the colour of the fibres and also the problems of breaking of the said fibres such as the hair. The composition and the process of the invention will also improve the physical properties of the hair, by durably reducing the volume of the hair and the frizziness effect.

In the text hereinbelow, the term "*at least one*" is equivalent to the term "*one or more*".

According to the present invention, a monoacid is an organic compound comprising only one acid radical.

According to a particular embodiment, the monoacid corresponds to formula (I) below, stereoisomers thereof, organic or mineral salts and the corresponding solvates: 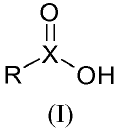 in which X represents a carbon atom (carboxylic acids), a group P-OH (phosphonic acids), a group P-H (phosphinic acids) or a group S=O (sulfonic acids), and R represents an alkyl, alkenyl, aryl or aralkyl radical with a linear, branched or cyclic alkyl group comprising from 1 to 20 carbon atoms, a linear, branched or cyclic alkenyl group comprising from 2 to 20 carbon atoms or an aryl or aralkyl group comprising from 6 to 20 carbon atoms, these groups being optionally substituted with one or more groups chosen from halogen atoms and hydroxyl, trifluoromethyl or C1-C4 alkoxy groups.

Among the monoacids of formula (I), mention may be made of glycolic (or hydroxyacetic) acid, lactic acid, 1-hydroxy-1-cyclopropanecarboxylic acid, 2-hydroxy-3-butenoic acid, 2-hydroxyisobutyric acid, 3-hydroxybutyric acid, 4-hydroxybutyric acid, 2-hydroxy-n-butyric acid, glyceric acid, 2-hydroxy-3-methylbutyric acid, 2-hydroxy-2-methylbutyric acid, 2-hydroxypentanoic acid, 3-hydroxypentanoic acid, 4-hydroxypentanoic acid, 2-hydroxyvaleric acid, 1-hydroxycyclohexanecarboxylic acid, mandelic acid, 2-hydroxy-3-methylvaleric acid, 2-trifluoromethyl-2-hydroxypropionic acid, hexahydroxymandelic acid, 2-hydroxyoctanoic acid, 3-phenyllactic acid, 3-hydroxymandelic acid, 4-hydroxymandelic acid, 2-hydroxynonanoic acid, 3-methoxymandelic acid, 4-methoxymandelic acid, 3-(4-hydroxyphenyl)lactic acid, β-chlorolactic acid, 1-cyclopentanol-1-carboxylic acid, 1,2-dihydroxycyclobutanecarboxylic acid, 2-ethyl-2-hydroxybutyric acid, α-hydroxyisocaproic acid, α-hydroxycaproic acid, 2-hydroxy-3,3-dimethylbutyric acid, gluconic acid, salicylic acid, 2-hydroxyhexanoic acid, 3-hydroxyhexanoic acid, 4-hydroxyhexanoic acid, 5-hydroxyhexanoic acid, 6-hydroxyhexanoic acid, hydroxypropylsulfonic acid, methanesulfonic acid, methanephosphonic acid, methylphosphinic acid, and also stereoisomers, organic or mineral salts and the corresponding solvates, alone or as a mixture.

In particular, the monoacids of formula (I) are chosen from glycolic acid, lactic acid, glyceric acid, gluconic acid, salicylic acid, 2-hydroxyisobutyric acid, 3-hydroxybutyric acid, 4-hydroxybutyric acid, 2-hydroxy-n-butyric acid, 2-hydroxyhexanoic acid, 3-hydroxyhexanoic acid, 4-hydroxyhexanoic acid, 5-hydroxyhexanoic acid, 6-hydroxyhexanoic acid, 2-hydroxypentanoic acid, 3-hydroxypentanoic acid, 4-hydroxypentanoic acid, hydroxypropylsulfonic acid and stereoisomers and organic or mineral salts, and the corresponding solvates, alone or as a mixture.

According to a preferred embodiment, the monoacid(s) are carboxylic acids.

According to a preferred variant of this embodiment, the carboxylic monoacid(s) are substituted on the radical R with at least one hydroxyl group. According to this variant, the radical R is preferably an alkyl, alkenyl, aryl or aralkyl radical with a linear, branched or cyclic alkyl group comprising from 1 to 20 carbon atoms, a linear, branched or cyclic alkenyl group comprising from 2 to 20 carbon atoms or an aryl group comprising from 6 to 20 carbon atoms, these groups being substituted with at least one hydroxyl radical, at least one of the hydroxyl radicals preferably being in the alpha or beta position relative to the acid group, and these groups being optionally substituted with one or more other groups chosen from halogen atoms and trifluoromethyl or C1-C4 alkoxy groups.

According to this variant, the monoacids of formula (I) are chosen from glycolic acid, lactic acid, glyceric acid, gluconic acid, salicylic acid, 2-hydroxyisobutyric acid, 2-hydroxy-n-butyric acid, 2-hydroxyhexanoic acid, 2-hydroxypentanoic acid and stereoisomers and organic or mineral salts, and the corresponding solvates, alone or as a mixture.

Even more preferentially, the monoacids of formula (I) are chosen from glycolic acid, lactic acid, glyceric acid, gluconic acid, salicylic acid and stereoisomers and organic or mineral salts, and the corresponding solvates, alone or as a mixture.

The amount of monoacids of the invention and/or of organic or mineral salts thereof, and/or of corresponding solvates thereof, in the composition that is useful for performing the invention may vary substantially. According to a particular embodiment, this amount ranges from 0.01% to 50%, preferably from 0.1% to 20% and even more preferentially from 1% to 15%, better from 5% to 15% and even better from 8% to 12% by weight relative to the total weight of the composition.

The composition that is useful in the process of the invention is aqueous-alcoholic. According to the invention, this composition comprises at least 5% of C1-C7 alcohol(s) relative to the total weight of the composition.

As alcohols present in the composition, mention may be made of monoalcohols and polyols comprising from 1 to 7 carbon atoms. By way of example, mention may be made of C₁-C₇ aliphatic monoalcohols, C₆-C₇ aromatic monoalcohols and C₃-C₇ polyols, used alone or as a mixture.

Preferably, the composition comprises one or more C₁-C₇ aliphatic alcohols. Preferably, the alcohol is a linear or branched monoalcohol comprising from 1 to 4 carbon atoms.

By way of example, mention may be made of ethanol, isopropanol, n-propanol, butanol, isobutanol or tert-butanol. Even more preferentially, the alcohol is chosen from ethanol and isopropanol. Better still, the alcohol is ethanol.

Preferably, the concentration of C1-C7 alcohols may range from 5% to 80%, better still from 10% to 70%, even better still from 20% to 60% and preferentially from 25% to 50% by weight relative to the total weight of the composition.

According to a particular embodiment, the composition comprises water in an amount ranging from 20% to 94.5% by weight relative to the total weight of the composition.

Preferably, the composition according to the invention does not comprise any colouring agent or any reducing agent.

According to the present invention, the term "*colouring agents*" means agents for colouring keratin fibres such as direct dyes, pigments or oxidation dye precursors (bases and couplers). If they are present, their content does not exceed 0.001% by weight relative to the total weight of the composition. This is because, at such a content, only the composition would be dyed, that is to say that no dyeing effect would be observed on the keratin fibres.

It is recalled that oxidation dye precursors, oxidation bases and couplers are colourless or sparingly coloured compounds, which, via a condensation reaction in the presence of an oxidizing agent, give a coloured species. With regard to direct dyes, these compounds are coloured and have a certain affinity for keratin fibres.

According to the present invention, the term "*reducing agent*" means an agent that is capable of reducing the disulfide bonds of the hair, such as compounds chosen from thiols, alkaline sulfites, hydrides and phosphines.

The composition that is useful in the process of the invention may comprise at least one thickener. For the purpose of the present invention, the term "thickener" means a compound/polymer which, when introduced at 1% in a pure aqueous solution or an aqueous-alcoholic solution containing 30% ethanol, and at pH = 7, makes it possible to achieve a viscosity of at least 100 cps and preferably of at least 500 cps, at 25°C and at a shear rate of 1 s⁻¹. This viscosity may be measured using a cone/plate viscometer (Haake R600 rheometer or the like). Preferably, the thickeners increase, by virtue of their presence, the viscosity of the compositions into which they are introduced by at least 50 cps and preferably 200 cps, at 25°C and at a shear rate of 1 s⁻¹.

The thickeners may be associative or non-associative polymers. The associative polymers comprise a C₁₀-C₃₀ fatty chain, whereas the non-associative thickening polymers do not comprise this type of fatty chain.

The thickening polymers of the invention may be anionic, cationic, nonionic or amphoteric. They are preferably nonionic.

Among the non-associative nonionic thickening polymers present, mention may be made of nonionic guar gums, biopolysaccharide gums of microbial origin, gums derived from plant exudates, celluloses, in particular hydroxypropylcelluloses or hydroxyethylcelluloses, and pectins, alone or as mixtures.

Among the associative polymers of nonionic type that may be mentioned are:
- (1) celluloses modified with groups comprising at least one fatty chain; examples that may be mentioned include:
   - hydroxyethylcelluloses modified with groups comprising at least one fatty chain, such as alkyl, arylalkyl or alkylaryl groups, or mixtures thereof, and in which the alkyl groups are preferably C₈-C₂₂, for instance the product Natrosol Plus Grade 330 CS® (C₁₆ alkyls) sold by the company Aqualon, or the product Bermocoll EHM 100® sold by the company Berol Nobel,
   - hydroxyethylcelluloses modified with alkylphenyl polyalkylene glycol ether groups, such as the product Amercell Polymer HM-1500® (polyethylene glycol (15) nonylphenyl ether) sold by the company Amerchol,
- (2) hydroxypropyl guars modified with groups comprising at least one fatty chain, such as the product Esaflor HM 22® (C₂₂ alkyl chain) sold by the company Lamberti, and the products RE210-18® (C₁₄ alkyl chain) and RE205-1® (C₂₀ alkyl chain) sold by the company Rhone-Poulenc,
- (3) copolymers of vinylpyrrolidone and of fatty-chain hydrophobic monomers; examples that may be mentioned include:
   - the products Antaron V216® or Ganex V216® (vinylpyrrolidone/hexadecene copolymer) sold by the company I.S.P.
   - the products Antaron V220® or Ganex V220® (vinylpyrrolidone/eicosene copolymer) sold by the company I.S.P.,
- (4) copolymers of C₁-C₆ alkyl methacrylates or acrylates and of amphiphilic monomers comprising at least one fatty chain, for instance the oxyethylenated methyl acrylate/stearyl acrylate copolymer sold by the company Goldschmidt under the name Antil 208®,
- (5) copolymers of hydrophilic methacrylates or acrylates and of hydrophobic monomers comprising at least one fatty chain, for instance the polyethylene glycol methacrylate/lauryl methacrylate copolymer,
- (6) polyurethane polyethers comprising in their chain both hydrophilic blocks usually of polyoxyethylenated nature and hydrophobic blocks, which may be aliphatic sequences alone and/or cycloaliphatic and/or aromatic sequences,
- (7) polymers with an aminoplast ether backbone containing at least one fatty chain, such as the Pure Thix® compounds sold by the company Sud-Chemie.

Preferably, the nonionic thickening polymers according to the invention are chosen from polysaccharides and associative polymers. The preferred nonionic thickening polymers are guar gums, hydroxyalkyl celluloses optionally modified with a hydrophobic group, such as hydroxyethylcelluloses, hydroxymethylcelluloses optionally modified with a hydrophobic group, and inulins optionally modified with a hydrophobic group.

Among the anionic thickening polymers, mention may be made of anionic associative polymers such as copolymers of acrylic or methacrylic acid and of acrylic or methacrylic esters with optionally oxyethylenated C8-C30 fatty alcohols, such as the polymers proposed by Lubrizol under the names Carbopol 1382 or Carbopol ETD 2020 or Pemulen TR1 or Pemulen TR2 and by Rohm & Haas under the name Aculyn 22.
The non-associative anionic thickening polymers are, for example, anionic polysaccharide polymers and particularly those belonging to the classes of celluloses and starches. By way of example, use may be made of carboxymethyl celluloses and salts thereof such as the product Blanose 7M8SF sold by the company Aqualon, carboxymethyl starches and salts thereof such as Primojel sold by the company DMV International, and starch phosphates (starch diphosphate or distarch phosphate) such as the products sold by the companies Dr Hauser or Akzo Nobel, respectively, under the names Rice Starch PO 4 and Structure Zea.

The amount of thickener in the composition used in the process of the invention may be between 0.1% and 10% and preferably between 0.5% and 5% relative to the total amount of the composition (percentage of active material).

The compositions that are useful in the process of the invention may be in any conventionally used galenical form. These compositions may be packaged in pump-action bottles or in aerosol containers, so as to apply the composition in vaporized (lacquer) form or in the form of a mousse. Such packaging forms are indicated, for example, when it is desired to obtain a spray or a mousse, for treating the hair. In these cases, the composition in aerosol form preferably comprises at least one propellant.

The composition of the invention may also comprise at least one common cosmetic ingredient, chosen in particular from oils; solid fatty substances and in particular C₈-C₄₀ esters; C₈-C₄₀ acids; fatty alcohols; surfactants; sunscreens; moisturizers; antidandruff agents; antioxidants; chelating agents; nacreous agents and opacifiers; plasticizers or coalescers; fillers; silicones other than the amino silicones and in particular polydimethylsiloxanes; polymeric or non-polymeric thickeners; gelling agents; emulsifiers; polymers, in particular conditioning or styling polymers; fragrances; basifying agents or acidifying agents; silanes; crosslinking agents. The composition can, of course, comprise several cosmetic ingredients appearing in the above list.

Depending on their nature and the purpose of the composition, the normal cosmetic ingredients can be present in normal amounts which can be easily determined by those skilled in the art and which can be, for each ingredient, between 0.01% and 80% by weight. A person skilled in the art will take care to choose the ingredients included in the composition, and also the amounts thereof, such that they do not harm the properties of the compositions of the present invention.

The pH of the composition is preferably less than 4 and preferably ranges from 1 to 3, better still from 1.5 to 3 and even better still from 1.7 to 3.

It may be adjusted to the desired value by means of acidifying and/or basifying agents usually used for treating keratin fibres, other than the organic monoacids of the invention.

The basifying agent may be chosen from mineral or organic or hybrid alkaline agents, or mixtures thereof.

The mineral alkaline agent(s) are preferably chosen from aqueous ammonia, alkali metal carbonates or bicarbonates such as sodium or potassium carbonates and sodium or potassium bicarbonates, sodium hydroxide or potassium hydroxide, or mixtures thereof.

The organic alkaline agent(s) are preferably chosen from organic amines with a pK_{b} at 25°C of less than 12, preferably less than 10 and even more advantageously less than 6. It should be noted that it is the pK_{b} corresponding to the function of highest basicity.

Hybrid compounds that may be mentioned include the salts of the amines mentioned previously with acids such as carbonic acid or hydrochloric acid. The organic alkaline agent(s) are chosen, for example, from amine derivatives such as alkanolamines, oxyethylenated and/or oxypropylenated ethylenediamines, and amines such as 1,3-diaminopropane, 1,3-diamino-2-propanol, spermine or spermidine.

The term "alkanolamine" means an organic amine comprising a primary, secondary or tertiary amine function, and one or more linear or branched C₁-C₈ alkyl groups bearing one or more hydroxyl radicals.

Sodium hydroxide is in particular suitable for use in the invention.

The acidifying agent may be chosen from mineral acids, for instance hydrochloric acid or phosphoric acid.

The process of the invention comprises a straightening/relaxing step. This step is generally a step of straightening the hair with an iron. Straightening with an iron is known from the prior art. It consists in straightening the hair with flat heating tongs, which are generally metallic. The straightening irons (or flat tongs) are generally used at a temperature of at least 150°C, preferably ranging from 150 to 250°C and preferentially ranging from 180 to 250°C. According to a preferred embodiment, the process of the invention is performed on curly or frizzy hair. The straightening/relaxing step is preferably performed using a straightening iron at a temperature of between 200 and 250°C.

The process of the invention may comprise other intermediate steps aimed at improving the straightening of the hair.

According to a particular embodiment, the process of the invention comprises the application of the composition of the invention to dry hair, and a time of contact of the composition with the hair of at least 10 minutes, preferably between 10 and 60 minutes and preferably between 15 and 45 minutes. After this leave-on time, straightening with a brush and with a hairdryer (blow-drying) is performed. The hair is then straightened with a straightening iron at a temperature of at least 150°C, preferably from 200 to 250°C.

The process of the invention may also comprise the application of other hair agents as a pre-treatment or post-treatment. In particular, it may comprise the application of a conditioning care product as a post-treatment.

According to another embodiment, the hair straightening process comprises a step of washing the hair and then of drying with a hairdryer before applying the composition of the invention. According to this particular embodiment, the steps described above are then encountered, such as the contact time of the composition, the blow-drying, the straightening with a straightening iron, the application of a conditioning agent and the rinsing, all these steps possibly being performed independently of each other. According to a particular embodiment, the straightening with the straightening iron is performed in several passes on the hair, in general 8 to 10 passes.

The process of the present invention is performed without a step of permanent reshaping based on a reducing agent. According to a particular embodiment, the process of the invention comprises the application of an aqueous-alcoholic composition comprising at least 5% of at least one C1-C7 alcohol and at least 5% of at least one organic monoacid, followed by a straightening/relaxing step using a straightening iron at a temperature of at least 180°C. According to this particular embodiment, the at least one organic monoacid is preferably chosen from glycolic acid, lactic acid, glyceric acid, gluconic acid, salicylic acid, 2-hydroxyisobutyric acid, 2-hydroxy-n-butyric acid, 2-hydroxyhexanoic acid, 2-hydroxypentanoic acid and stereoisomers and organic or mineral salts, and the corresponding solvates, alone or as a mixture.

### Examples

The following compositions were prepared (the percentages indicated are expressed on a weight basis):

| **Compositions** | **1** | **2** | **3** | **4** |
|---|---|---|---|---|
| Glycolic acid | 10 | | 10 | |
| Lactic acid | | 10 | | 10 |
| Ethanol | 30 | 30 | | |
| Isopropanol | | | 30 | 30 |
| Sodium hydroxide qs pH | 2.1 | 2.1 | 2.1 | 2.1 |
| Water qs | 100 | 100 | 100 | 100 |

| **Composition** | **5** | **6** | **7** | **8** |
|---|---|---|---|---|
| Glycolic acid | 10 | | 10 | |
| Lactic acid | | 10 | | 10 |
| Ethanol | 30 | 30 | 30 | 30 |
| Potassium hydroxide qs pH | 2.1 | 2.1 | | |
| Triethanolamine qs pH | | | 2.1 | 2.1 |
| Water qs | 100 | 100 | 100 | 100 |

**Control composition:** The control compositions 1' to 8' were prepared on the model of compositions 1 to 8 in which the alcohol was replaced with water.

Compositions 1 to 8 and 1' to 8' are applied to natural curly hair (6 to 8 curls) 15 cm long, at a rate of 1 g per 2 g of hair. After 15 minutes, the hair is dried with a hairdryer (blow-drying) and then straightened by treatment with flat tongs heated to 210°C (10 passes per half-lock). They are subsequently shampooed to examine the persistence of the straightening effects and the modification of the mechanical and cosmetic properties of the fibres.

The straightening obtained with compositions 1 to 8 is equivalent to that obtained with the corresponding control compositions 1' to 8' in terms of straightening performance (curl relaxation), but, with the process of the invention, straightening that remains persistent after washing of the locks is obtained.

## Claims

1. Hair treatment process comprising the application to the hair of an aqueous-alcoholic composition comprising at least 5% of at least one C1-C7 alcohol and at least one organic monoacid, followed by a straightening/relaxing step using a straightening iron at a temperature of at least 100°C, the process being performed without a step of permanent reshaping based on a reducing agent.

2. Process according to Claim 1, in which the monoacid corresponds to formula (I) below, stereoisomers thereof, organic or mineral salts and the corresponding solvates: 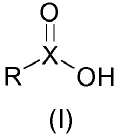 in which X represents a carbon atom, a group P-OH, a group P-H or a group S=O, and R represents an alkyl, alkenyl, aryl or aralkyl radical with a linear, branched or cyclic alkyl group comprising from 1 to 20 carbon atoms, a linear, branched or cyclic alkenyl group comprising from 2 to 20 carbon atoms or an aryl or aralkyl group comprising from 6 to 20 carbon atoms, these groups being optionally substituted with one or more groups chosen from halogen atoms and hydroxyl, trifluoromethyl or C1-C4 alkoxy groups.

3. Process according to Claim 1 or 2, in which the monoacid(s) are carboxylic acids.

4. Process according to any one of the preceding claims, in which the carboxylic monoacid(s) are chosen from the compounds of formula (I) in which X represents a carbon atom, the radical R is an alkyl, alkenyl or aralkyl radical with a linear, branched or cyclic alkyl group comprising from 1 to 20 carbon atoms, a linear, branched or cyclic alkenyl group comprising from 2 to 20 carbon atoms or an aryl or aralkyl group comprising from 6 to 20 carbon atoms, these groups being substituted with at least one hydroxyl radical, at least one of the hydroxyl radicals preferably being in the alpha or beta position relative to the acid group, and these groups being optionally substituted with one or more other groups chosen from halogen atoms and trifluoromethyl or C1-C4 alkoxy groups.

5. Process according to any one of the preceding claims, in which the monoacids are chosen from glycolic acid, lactic acid, glyceric acid, gluconic acid, salicylic acid, 2-hydroxyisobutyric acid, 2-hydroxy-n-butyric acid, 2-hydroxyhexanoic acid, 2-hydroxypentanoic acid and stereoisomers and organic or mineral salts, and the corresponding solvates, alone or as a mixture and preferably among glycolic acid, lactic acid, glyceric acid, gluconic acid, salicylic acid and stereoisomers and organic or mineral salts, and the corresponding solvates, alone or as a mixture.

6. Process according to any one of the preceding claims, in which the amount of monoacids and/or of organic or mineral salts thereof, and/or of corresponding solvates thereof, ranges from 0.01% to 50%, preferably from 0.1% to 20% and even more preferentially from 1% to 15% by weight, better from 5% to 15% and even better from 8% to 12% relative to the total weight of the composition.

7. Process according to any one of the claims, in which the amount of C1-C7 alcohol(s) ranges from 5% to 80%, better still from 10% to 70%, even better still from 20% to 60% and preferentially from 25% to 50% by weight relative to the total weight of the composition.

8. Process according to any one of the preceding claims, in which the C1-C7 alcohol(s) are chosen from C₁-C₇ aliphatic monoalcohols, C₆-C₇ aromatic monoalcohols and C₃-C₇ polyols.

9. Process according to any one of the preceding claims, in which the C1-C7 alcohol(s) are chosen from linear or branched monoalcohols comprising from 1 to 4 carbon atoms, in particular from ethanol and isopropanol.

10. Process according to any one of the preceding claims, in which the C1-C7 alcohol is ethanol.

11. Process according to any one of the preceding claims, in which the composition comprises water in an amount ranging from 20% to 94.5% by weight relative to the total weight of the composition.

12. Process according to any one of the claims, in which the composition comprises at least one thickener.

13. Process according to any one of the preceding claims, in which the composition has a pH of less than 4, and preferably ranging from 1 to 3, better still from 1.5 to 3 and even better still from 1.7 to 3.

14. Process according to any one of the preceding claims, in which the hair straightening/relaxing step is performed at a temperature ranging from 150 to 250°C, preferably ranging from 180 to 250°C.

15. Process according to any one of the preceding claims, in which the composition is applied to dry hair, and, after a leave-on time of at least 10 minutes and preferably between 15 and 45 minutes, the hair is then dried using a brush, and is then straightened with a straightening iron at a temperature of at least 150°C, preferably ranging from 200 to 250°C.

## Patentansprüche

1. Haarbehandlungsverfahren, umfassend Aufbringen einer wässrig-alkoholischen Zusammensetzung, die wenigstens 5 % an wenigstens einem C1-C7-Alkohol und wenigstens eine organische Monosäure umfasst, auf das Haar, gefolgt von einem Glättungs/Entkräuselungsschritt unter Verwendung eines Glätteisens bei einer Temperatur von wenigstens 100 °C, wobei das Verfahren ohne einen Schritt des dauerhaften Umformens auf der Basis eines Reduktionsmittels durchgeführt wird.

2. Verfahren gemäß Anspruch 1, wobei die Monosäure der nachstehenden Formel (I), Stereoisomeren davon, organischen oder Mineralsalzen und den entsprechenden Solvaten entspricht: 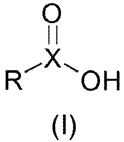 wobei X ein Kohlenstoffatom, eine Gruppe P-OH, eine Gruppe P-H oder eine Gruppe S=O darstellt und R einen Alkyl-, Alkenyl-, Aryl- oder Aralkylrest mit einer linearen, verzweigten oder cyclischen Alkylgruppe umfassend von 1 bis 20 Kohlenstoffatome, einer linearen, verzweigten oder cyclischen Alkenylgruppe umfassend von 2 bis 20 Kohlenstoffatome oder einer Aryl- oder Aralkylgruppe umfassend von 6 bis 20 Kohlenstoffatomen darstellt, wobei diese Gruppen gegebenenfalls mit einer oder mehreren Gruppen ausgewählt aus Halogenatomen und Hydroxy-, Trifluormethyl- oder C1-C4-Alkoxygruppen substituiert sind.

3. Verfahren gemäß Anspruch 1 oder 2, wobei die Monosäure(n) Carbonsäuren sind.

4. Verfahren gemäß einem der vorstehenden Ansprüche, wobei die Monocarbonsäure(n) ausgewählt sind aus den Verbindungen der Formel (I), wobei X ein Kohlenstoffatom darstellt, der Rest R einen Alkyl-, Alkenyl- oder Aralkylrest mit einer linearen, verzweigten oder cyclischen Alkylgruppe umfassend von 1 bis 20 Kohlenstoffatome, einer linearen, verzweigten oder cyclischen Alkenylgruppe umfassend von 2 bis 20 Kohlenstoffatome oder einer Aryl- oder Aralkylgruppe umfassend von 6 bis 20 Kohlenstoffatomen darstellt, wobei diese Gruppen mit wenigstens einem Hydroxyrest substituiert sind, wobei wenigstens einer der Hydroxyreste vorzugsweise an der alpha- oder beta-Position bezogen auf die Säuregruppe angeordnet ist und diese Gruppen gegebenenfalls mit einer oder mehreren anderen Gruppen ausgewählt aus Halogenatomen und Trifluormethyl- oder C1-C4-Alkoxygruppen substituiert sind.

5. Verfahren gemäß einem der vorstehenden Ansprüche, wobei die Monosäuren ausgewählt sind aus Glycolsäure, Milchsäure, Glycerinsäure, Gluconsäure, Salicyclsäure, 2-Hydroxyisobuttersäure, 2-Hydroxy-n-buttersäure, 2-Hydroxyhexansäure, 2-Hydroxypentansäure und Stereoisomeren und organischen oder Mineralsalzen und den entsprechenden Solvaten, allein oder als Gemisch, und vorzugsweise aus Glycolsäure, Milchsäure, Glycerinsäure, Gluconsäure, Salicyclsäure und Stereoisomeren und organischen oder Mineralsalzen und den entsprechenden Solvaten, allein oder als Gemisch.

6. Verfahren gemäß einem der vorstehenden Ansprüche, wobei die Menge an Monosäuren und/oder organischen oder Mineralsalzen davon und/oder entsprechenden Solvaten davon in dem Bereich von 0,01 Gew.-% bis 50 Gew.-%, vorzugsweise von 0,1 Gew.-% bis 20 Gew.-% und noch bevorzugter von 1 Gew.-% bis 15 Gew.-%, besser von 5 Gew.-% bis 15 Gew.-% und noch besser von 8 Gew.-% bis 12 Gew.-%, bezogen auf das Gesamtgewicht der Zusammensetzung, liegt.

7. Verfahren gemäß einem der vorstehenden Ansprüche, wobei die Menge an C1-C7-Alkohol(en) in dem Bereich von 5 Gew.-% bis 80 Gew.-%, besser von 10 Gew.-% bis 70 Gew.-%, noch besser von 20 Gew.-% bis 60 Gew.-% und vorzugsweise von 25 Gew.-% bis 50 Gew.-%, bezogen auf das Gesamtgewicht der Zusammensetzung, liegt.

8. Verfahren gemäß einem der vorstehenden Ansprüche, wobei die C1-C7-Alkohol(e) ausgewählt sind aus aliphatischen C₁-C₇-Monoalkoholen, aromatischen C₆-C₇-Monoalkoholen und C₃-C₇-Polyolen.

9. Verfahren gemäß einem der vorstehenden Ansprüche, wobei die C1-C7-Alkohol(e) ausgewählt sind aus linearen oder verzweigten Monoalkoholen, die von 1 bis 4 Kohlenstoffatome umfassen, insbesondere aus Ethanol und Isopropanol.

10. Verfahren gemäß einem der vorstehenden Ansprüche, wobei der C1-C7-Alkohol Ethanol ist.

11. Verfahren gemäß einem der vorstehenden Ansprüche, wobei die Zusammensetzung Wasser in einer Menge in dem Bereich von 20 Gew.-% bis 94,5 Gew.-%, bezogen auf das Gesamtgewicht der Zusammensetzung, umfasst.

12. Verfahren gemäß einem der vorstehenden Ansprüche, wobei die Zusammensetzung wenigstens ein Verdickungsmittel umfasst.

13. Verfahren gemäß einem der vorstehenden Ansprüche, wobei die Zusammensetzung einen pH-Wert von weniger als 4 und vorzugsweise in dem Bereich von 1 bis 3, besser von 1,5 bis 3 und noch besser von 1,7 bis 3, aufweist.

14. Verfahren gemäß einem der vorstehenden Ansprüche, wobei der Haarglättungs/entkräuselungsschritt bei einer Temperstur in dem Bereich von 150 bis 250 °C, vorzugsweise in dem Bereich von 180 bis 250 °C, durchgeführt wird.

15. Verfahren gemäß einem der vorstehenden Ansprüche, wobei die Zusammensetzung auf trockenes Haar aufgebracht wird und nach einer Verweilzeit von wenigstens 10 Minuten und vorzugsweise zwischen 15 und 45 Minuten das Haar unter Verwendung einer Bürste getrocknet und dann mit einem Glätteisen bei einer Temperatur von wenigstens 150 °C, vorzugsweise in dem Bereich von 200 bis 250 °C, geglättet wird.

## Revendications

1. Procédé de traitement des cheveux comprenant l'application sur les cheveux d'une composition hydroalcoolique comprenant au moins 5% d'au moins un alcool en C1-C7 et au moins un monoacide organique, suivie d'une étape de lissage/défrisage au moyen d'un fer à lisser à une température d'au moins 100°C, le procédé étant mis en oeuvre sans étape de remodelage permanent se basant sur un agent réducteur.

2. Procédé selon la revendication 1, dans lequel le monoacide répond à la formule (I) suivante, ses stéréoisomères, les sels organiques ou minéraux et les solvates correspondants : 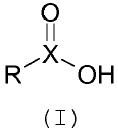 dans laquelle X représente un atome de carbone, un groupe P-OH, un groupe P-H ou un groupe S=O et R représente un radical alkyle, alcényle, aryle ou aralkyle avec un groupe alkyle linéaire, ramifié ou cyclique qui comprend de 1 à 20 atomes de carbone, un groupe alcényle linéaire, ramifié ou cyclique qui comprend de 2 à 20 atomes de carbone ou un groupe aryle ou aralkyle qui comprend de 6 à 20 atomes de carbone, ces groupes étant éventuellement substitués par un ou plusieurs groupements choisis parmi les atomes d'halogène et les groupements hydroxyle, trifluorométhyle ou alcoxy C1-C4.

3. Procédé selon la revendication 1 ou 2, dans lequel le ou les monoacides sont des acides carboxyliques.

4. Procédé selon l'une quelconque des revendications précédentes, dans lequel le ou les monoacides carboxyliques sont choisis parmi les composés de formule (I) dans laquelle X représente un atome de carbone, le radical R est un radical alkyle, alcényle ou aralkyle avec un groupe alkyle linéaire, ramifié ou cyclique qui comprend de 1 à 20 atomes de carbone, un groupe alcényle linéaire, ramifié ou cyclique qui comprend de 2 à 20 atomes de carbone ou un groupe aryle ou aralkyle qui comprend de 6 à 20 atomes de carbone, ces groupes étant substitués par au moins un radical hydroxyle, un au moins des radicaux hydroxyle étant de préférence situé en position alpha ou bêta du groupe acide et ces groupes étant éventuellement substitués par un ou plusieurs autres groupements choisis parmi les atomes d'halogène et les groupements trifluorométhyle ou alcoxy C1-C4.

5. Procédé selon l'une quelconque des revendications précédentes, dans lequel les monoacides sont choisis parmi l'acide glycolique, l'acide lactique, l'acide glycérique, l'acide gluconique, l'acide salicylique, l'acide 2-hydroxyisobutyrique, l'acide 2-hydroxy-n-butyrique, l'acide 2-hydroxyhexanoique, l'acide 2-hydroxypentanoique et les stéréoisomères, les sels organiques ou minéraux et les solvates correspondants, seuls ou en mélange, et de préférence parmi l'acide glycolique, l'acide lactique, l'acide glycérique, l'acide gluconique, l'acide salicylique et les stéréoisomères, les sels organiques ou minéraux et les solvates correspondants, seuls ou en mélange.

6. Procédé selon l'une quelconque des revendications précédentes, dans lequel la quantité de monoacides et/ou de leurs sels organiques ou minéraux, et/ou de leurs solvates correspondants varie de 0,01 à 50%, de préférence de 0,1 à 20% encore plus préférablement de 1 à 15% en poids, mieux de 5 à 15% et encore mieux de 8 à 12% par rapport au poids total de la composition.

7. Procédé selon l'une quelconque des revendications, dans lequel la quantité d'alcool(s) en C1-C7 varie de 5 à 80%, mieux de 10 à 70%, encore mieux de 20 à 60%, préférentiellement de 25 à 50% en poids par rapport au poids total de la composition.

8. Procédé selon l'une quelconque des revendications précédentes, dans lequel le ou les alcools en C1-C7 sont choisis parmi les monoalcools aliphatiques en C₁-C₇, les monoalcools aromatiques en C₆-C₇ et les polyols en C₃-C₇.

9. Procédé selon l'une quelconque des revendications précédentes, dans lequel le ou les alcools en C1-C7 sont choisis parmi les monoalcools linéaires ou ramifiés comprenant de 1 à 4 atomes de carbone, en particulier parmi l'éthanol ou l'isopropanol.

10. Procédé selon l'une quelconque des revendications précédentes, dans lequel l'alcool en C1-C7 est l'éthanol.

11. Procédé selon l'une quelconque des revendications précédentes, dans lequel la composition comprend de l'eau en quantité allant de 20 à 94,5% en poids par rapport au poids total de la composition.

12. Procédé selon l'une quelconque des revendications, dans lequel la composition comprend au moins un épaississant.

13. Procédé selon l'une quelconque des revendications précédentes, dans lequel la composition présente un pH inférieur à 4, et variant de préférence de 1 à 3, mieux de 1,5 à 3, encore mieux de 1,7 à 3.

14. Procédé selon l'une quelconque des revendications précédentes, dans lequel l'étape de lissage/défrisage des cheveux est mise en oeuvre à une température allant de 150 à 250°C, de préférence allant de 180 à 250°C.

15. Procédé selon l'une quelconque des revendications précédentes, dans lequel la composition est appliquée sur cheveux secs, après un temps de pose d'au moins 10 min, de préférence entre 15 et 45 min, les cheveux sont alors séchés à l'aide d'une brosse, et ensuite lissés au fer à lisser à une température d'au moins 150°C, de préférence variant de 200 à 250°C.
